(19) Europäisches Patentamt
European Patent Office
Office européen des brevets

(11) EP 1 460 052 A1

(12) **EUROPÄISCHE PATENTANMELDUNG**

(43) Veröffentlichungstag:
**22.09.2004 Patentblatt 2004/39**

(51) Int Cl.[7]: **C07C 41/56**, C07C 41/58

(21) Anmeldenummer: **04100262.7**

(22) Anmeldetag: **26.01.2004**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **21.03.2003 DE 10312562**

(71) Anmelder: **Degussa AG**
**40474 Düsseldorf (DE)**

(72) Erfinder:
- **Ruwwe, Johannes**
  **53859, Niederkassel (DE)**
- **Brühl, Jutta**
  **46282, Dorsten (DE)**
- **Osterholt, Clemens**
  **46286, Dorsten (DE)**

(54) **Verfahren zur kontinuierlichen Herstellung von Acetalen von alpha, beta-Dicarbonylverbindungen**

(57) Die vorliegende Erfmdung betrifft ein Verfahren zur Herstellung von Acetalen von α,β-Dicarbonylverbindungen der allgemeinen Formel

$$(R''O)_2CRCR'(OR'')_2$$

wobei man diese durch kontinuierliche Reaktion von α, β-Dicarbonylverbindungen R-CO-CO-R' mit Alkoholen R"OH oder HO-X-OH im Gegenstrom erhält.

EP 1 460 052 A1

## Beschreibung

Die vorliegende Erfindung betrifft ein Verfahren zur kontinuierlichen Herstellung von Acetalen von α,β-Dicarbonylverbindungen.

Acetale von α,β-Dicarbonylverbindungen des Typs $(R''O)_2CRCR'(OR'')_2$ (I) wie zum Beispiel die des Glyoxals können als Vernetzer (cross-linker) für Faserwerkstoffe aus Polyvinylalkohol oder bei der Verarbeitung von Cellulose, Papier, Leder und Baumwolle eingesetzt werden. Solche Verbindungen können ebenfalls als Hilfsmittel in der Aufbereitung von Erzen verwendet werden.
Eine Anwendung von Acetalen des Glyoxals als Zusatzstoff in Diesel und Benzin ist in DE 199 20 270 beschrieben.
Eine weitere Anwendung ist in WO 02 20 446 angegeben, die eine elektrochemische Darstellung von Orthocarbonsäureestern aus Acetalen von α,β-Dicarbonylverbindungen beschreibt.
Acetale von α,β-Dicarbonylverbindungen können durch Reaktion von α,β-Dicarbonylverbindungen mit Alkoholen hergestellt werden. Diese Reaktionen sind üblicherweise säurekatalysiert.

$$R\text{-}CO\text{-}CO\text{-}R' + 4\ R''OH \rightarrow (R''O)_2CRCR'(OR'')_2 + 2\ H_2O$$

Für das Beispiel des Glyoxals (R, R' = H) sind solche Verfahren in diskontinuierlicher Fahrweise zum Beispiel in US 2 360 959, GB 559 362, US 2 360 957, US 2 361 456, SU 434 078, US 5 191 127 sowie in J. Org. Chem. (1972), 37, 1276; J. Org. Chem. (1973), 38, 556; J. Org. Chem. (1974), 39, 1772 und Recueil des Travaux Chimique des Pays-Bas (1990), 109, 15 beschrieben. Das entstehende Reaktionswasser sowie das als Lösemittel eingesetzte Wasser werden durch Co-destillation mit dem eingesetzten Alkohol beziehungsweise durch azeotrope Destillation mit einem zusätzlichen Stoff (Schleppmittel) entfernt.
Der Nachteil der beschriebenen Verfahren liegt in den schlechten Raum-Zeit-Ausbeuten, den teilweise mäßigen Ausbeuten insbesondere bei der Verwendung von niedrig siedenden Alkoholen wie Methanol oder Ethanol (R" = Me, Et) und teilweise unbefriedigender Selektivität.
Analoge Reaktionen mit höheren homologen α,β-Dicarbonylverbindungen werden zum Beispiel in Bull. Soc. Chim. Fr. (1976), 3 - 4, 601 oder J. Chem. Soc., Perkin Trans. II (1972), 4, 357 beschrieben. In US 2 421 559 und EP 0 704 422 werden genannte Verbindungen I (R = H, R' = Methyl, R" = Methyl, Butyl) als unerwünschte Nebenprodukte bei der Synthese von Methylglyoxaldialkylacetalen $(Me\text{-}CO\text{-}CH(OR'')_2$ genannt.

Die Herstellung der Acetale von α,β-Dicarbonylverbindungen des Typs $(R''O)_2CRCR'(OR'')_2$ durch Umsetzung von α,β-Dicarbonylverbindungen mit Alkoholen durch saure Katalyse kann durch Zugabe von wasserentziehenden Mitteln beschleunigt werden. Dieses hat jedoch den Nachteil, dass zusätzliche Stoffe, wie zum Beispiel Trimethylsilylchlorid (Synthesis (1983), 203) oder Trimethylorthoformiat (J. Org. Chem. (1996), 61, 3897), in den Prozess mit aufgenommen werden müssen, was die Wirtschaftlichkeit eines solchen Verfahrens erheblich mindert.
Eine weitere Methode zur Darstellung von Acetalen von (α, β-Dicarbonylverbindungen, speziell des Glyoxaltetraethylacetals (I; R, R' = H, R" = Ethyl) besteht in der Umsetzung von Ethanol mit NOCl, wie es in US 3 130 234 beschrieben ist. Der Nachteil dieses Verfahrens besteht darin, dass lediglich dieses beschriebene Derivat zugänglich ist und dass mit dem schlecht verfügbaren Nitrosylchlorid umgegangen werden muss.

Aufgabe der vorliegenden Erfindung war daher die Angabe eines Verfahrens zur Herstellung von Acetalen von α,β-Dicarbonylverbindungen, welches insbesondere im technischen Maßstab unter ökonomischen und ökologischen Gesichtspunkten vorteilhaft anzuwenden ist und darüber hinaus die Nachteile der Verfahren des Standes der Technik vermeidet.

Die Aufgabe wird gelöst, indem in einem kontinuierlich geführten Verfahren Verbindungen der allgemeinen Formel (I) hergestellt werden

$$(R''O)_2CRCR'(OR'')_2 \qquad (I),$$

worin
R und R' unabhängig voneinander für H, (C1-C8)-Alkyl, (C3-C8)-cyclo-Alkyl, (C2-C8)-Alkenyl, (C2-C8)-Alkinyl, (C6-C18)-Aryl und
R" unabhängig voneinander für (C1-C8-Alkyl), (C3-C8)-cyclo-Alkyl, (C2-C8)-Alkenyl, (C2-C8)-Alkinyl oder aber für eine Kette X, wobei X die Bedeutung einer (C2-C12)-Alkylenkette oder (C2-C12)-Alkenylenkette hat, die die beiden Sauerstoffatome des α-Kohlenstoffatoms und/oder die beiden Sauerstoffatome des β-Kohlenstoffatoms miteinander verknüpft, (Acetale (Ia))

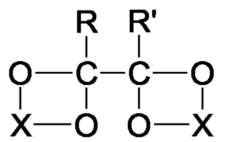

(Ia)

steht,
indem man Verbindungen des Typs R-CO-CO-R', wobei R und R' die zuvor genannten Bedeutungen haben, mit einwertigen Alkoholen des Typs R"OH oder zweiwertigen Alkoholen des Typs HO-X-OH (Glykole, Diole), wobei R" und X die zuvor genannten Bedeutungen haben, in einer Apparatur, die eine kontinuierliche Reaktion im Gegenstromprinzip zulässt, zur Reaktion bringt.
Überraschenderweise können die erwünschten Produkte in guten Ausbeuten und Reinheiten mit guten Raum-Zeit-Ausbeuten isoliert werden.

Gegenstand der Erfindung ist daher ein Verfahren zur Herstellung der Verbindungen der allgemeinen Formel (I) $(R"O)_2CRCR'(OR")_2$ gemäß Anspruch 1.

Die Reaktion wird vorteilhafterweise so gefahren, dass eine α,β-Dicarbonylverbindung R-CO-CO-R', entweder als Reinstoff oder in Form einer Lösung in Wasser oder einem organischen Lösemittel wie zum Beispiel einem Alkohol, Amid, Ester, Ether, aliphatischen, aromatischen Kohlenwasserstoff oder einem halogenierten Kohlenwasserstoff, in eine Gegenstromapparatur wie zum Beispiel eine Reaktionskolonne, einen Dünnschichtverdampfer, einen Fallfilmverdampfer oder eine Rührkesselkaskade eingespeist wird, während gleichzeitig ein einwertiger Alkohol R"OH oder zweiwertiger Alkohol HO-X-OH vorzugsweise in gasförmiger Form in diese Apparatur eingespeist wird, derart, dass der aufsteigende Dampf des Alkohols R"OH beziehungsweise HO-X-OH der ablaufenden α,β-Dicarbonylverbindung R-CO-CO-R' beziehungsweise deren Lösung in einem Lösemittel entgegen strömt.

Es ist jedoch auch möglich, den Alkohol R"OH oder HO-X-OH in flüssiger Form einzuspeisen und ihn erst in der Apparatur zu verdampfen.

Je nach Flüchtigkeit der Zielverbindung $(R"O)_2CRCR'(OR")_2$ und dem Siedepunkt des eingesetzten Alkohols R"OH oder HO-X-OH wird das Produkt mit dem Destillat oder mit dem Sumpfablauf der eingesetzten Apparatur erhalten und kann mittels Destillation aufgereinigt werden.

Die Reaktion kann in Gegenwart oder in Abwesenheit eines Katalysators erfolgen, bevorzugt in Gegenwart eines Katalysators, insbesondere in Gegenwart einer Protonen- oder einer LewisSäure. Dieser Katalysator kann entweder in gasförmiger Form mit dem eingesetzten einwertigen Alkohol R"OH oder dem zweiwertigen Alkohol HO-X-OH oder aber in gelöster Form zusammen mit der eingesetzten α,β-Dicarbonylverbindung R-CO-CO-R' zugeführt werden. Mögliche Katalysatoren sind zum Beispiel Sulfonsäuren, Schwefelsäure, Salzsäure, Hydrogensulfate oder Carbonsäuren. Ebenfalls ist es möglich, heterogene Katalysatoren, wie zum Beispiel Ionentauscher, Montmorillonite, oder saure Oxide einzusetzen, die entweder an der Wandung der Apparatur befestigt sind oder aber in Form einer katalytisch aktiven Packung eingebracht werden.

Die Reaktionstemperatur beträgt 20 bis 250 °C, vorzugsweise 50 bis 200 °C, besonders bevorzugt 60 bis 150 °C. Es ist möglich, die Reaktion ohne externe Kühlung oder Beheizung durchzuführen, wobei die Temperatur sich über die Siedetemperatur des eingesetzten Alkohols R"OH oder HO-X-OH beim eingestellten Druck und der Temperatur der entgegenströmenden α,β-Dicarbonylverbindung R-CO-CO-R' beziehungsweise deren Lösung einstellt. Es ist ebenfalls möglich die verwendete Apparatur zu kühlen oder zu heizen. Weiterhin können die zugeführten Stoffströme ebenfalls gekühlt oder beheizt werden.

Die Reaktion wird bei Drucken von 20 mbar bis 20 bar, vorzugsweise von 500 mbar bis 5 bar durchgeführt. Besonders bevorzugt wird der Druckbereich von 1 bis 3 bar.

Das molare Verhältnis der aufgegebenen Stoffströme zwischen der α,β-Dicarbonylverbindung R-CO-CO-R' und dem einwertigen Alkohol R"OH kann zwischen 1 : 2 und 1 : 2000 liegen, bevorzugt sind Verhältnisse zwischen 1 : 4 und 1 : 80, ganz besonders bevorzugt sind Verhältnisse zwischen 1 : 8 und 1 : 20.

Das molare Verhältnis der aufgegebenen Stoffströme zwischen der α,β-Dicarbonylverbindung R-CO-CO-R' und dem zweiwertigen Alkohol HO-X-OH kann zwischen 1 : 1 und 1 : 1000 liegen, bevorzugt sind Verhältnisse zwischen 1 : 2 und 1 : 40, ganz besonders bevorzugt sind Verhältnisse zwischen 1 : 4 und 1 : 10.

Als bevorzugte α,β-Dicarbonylverbindungen werden Verbindungen der allgemeinen Formel R-CO-CO-R', bei denen R und R' unabhängig voneinander für H, (C1-C8)-Alkyl, (C3-C8)-cyclo-Alkyl, (C2-C8)-Alkenyl, (C2-C8)-Alkinyl oder (C6-C18)-Aryl steht, eingesetzt. Bevorzugt sind jedoch Verbindungen, bei denen R und R' unabhängig voneinander für H oder (C1-C8)-Alkyl stehen. Ganz besonders bevorzugt sind Verbindungen, bei denen R und R' für H oder Methyl stehen, wobei R und R' unabhängig voneinander sind, insbesondere also die Verbindungen Diacyl, Glyoxal oder Methylglyoxal.

Als bevorzugte Alkohole werden einwertige Alkohole der allgemeinen Formel R"OH, bei denen R" für (C1-C8-Alkyl), (C3-C8)-cyclo-Alkyl, (C2-C8)-Alkenyl oder (C2-C8)-Alkinyl steht, eingesetzt. Ebenfalls können als Alkohole zweiwertige Alkohole der allgemeinen Formel HO-X-OH eingesetzt werden, wobei X für eine (C2-C12)-Alkylenkette oder eine (C2-C12)-Alkenylenkette steht. Insbesondere bevorzugte Verbindungen sind Alkohole der Formel R"OH, bei denen R" für (C1-C8-Alkyl) steht, ganz besonders bevorzugt sind Methanol, Ethanol, n-Propanol, iso-Propanol, n-Butanol, 2-Butanol und 2-Methyl-1-propanol. Es können auch Mischungen von Alkoholen eingesetzt werden.

Die nach dem beschriebenen Verfahren anfallenden Destillate und Sumpfabläufe enthalten in Abhängigkeit von den physikalischen Eigenschaften der Produkte und Edukte in variierenden Konzentrationen folgende Verbindungen:
$(R''O)_2CRCR'(OR'')_2$ (I), $(R''O)_2CR$-CO-R', R"OH beziehungsweise HO-X-OH, Wasser, eventuell nicht umgesetzte α, β-Dicarbonylverbindung R-CO-CO-R', für den Fall R, R' = H (Glyoxal) eventuell oligomere Nebenprodukte sowie 2-Hydroxyessigsäuremethylester und 2-Hydroxyessigsäure, aus denen die Zielverbindung durch kontinuierliche oder diskontinuierliche Destillation isoliert werden kann.

Um besonders wirtschaftliche Prozesse realisieren zu können, sollten der aus diesen Destillationen zurück gewonnene Alkohol R"OH beziehungsweise HO-X-OH und die halbacetalisierte Verbindung $(R''O)_2CR$-CO-R' in den Prozess zurückgeführt werden, wobei der Alkohol R"OH beziehungsweise HO-X-OH wiederum in gasförmiger Form in die verwendeten Apparatur eingespeist wird, und das halbacetalisierte Produkt $(R''O)_2CR$-CO-R' zusammen mit der α,β-Dicarbonylverbindung R-CO-CO-R' derart in die Apparatur eingespeist wird, dass ein Gegenstromprinzip erhalten bleibt.

Bevorzugt sind dabei Prozesse, die derart gefahren werden, dass an einer weiter unten liegenden Eintrittsstelle einer vertikalen Gegenstromapparatur ein einwertiger Alkohol R"OH oder ein zweiwertiger Alkohol HO-X-OH in gasförmiger Form eindosiert wird und gleichzeitig an einer weiter oben liegenden Eintrittsstelle dieser Apparatur eine α,β-Dicarbonylverbindung R-CO-CO-R' in Substanz oder in Lösung eingefahren wird, so dass der anfallende Sumpfablauf das gewünschte Acetal $(R''O)_2CRCR'(OR'')_2$ (I) und gegebenenfalls das halb acetalisierte Produkt $(R''O)_2CR$-CO-R' sowie den eingesetzten Alkohol R"OH oder HO-X-OH enthält, während das Destillat nur aus dem Alkohol R"OH oder HO-X-OH und Wasser besteht. Der Sumpfablauf und das Destillat werden getrennt in weiteren Destillationsapparaten aufgearbeitet. Soweit eine ausreichende Aufreinigung möglich ist, werden neben dem Zielprodukt noch die Verbindungen $(R''O)_2CR$-CO-R' und R"OH beziehungsweise HO-X-OH in möglichst reiner Form zurückgewonnen und in den Prozess zurückgeführt.

Ganz besonders bevorzugt sind Verfahren, bei denen neben der Herstellung des Produktes $(R''O)_2CRCR'(OR'')_2$ (I) auch die Aufarbeitung des anfallenden Sumpfablaufes und des Destillates mittels eines kontinuierlichen Prozesses vorgenommen wird.

Die folgenden Beispiele sollen den Gegenstand der Erfindung näher erläutern:

<u>Beispiel 1</u> (erfindungsgemäß):

Darstellung von 1,1,2,2-Tetramethoxyethan

In eine beheizbare 1m-Kolonne (Durchmesser 29mm) mit Drahtgeflechteinsätzen wurde am Kopf eine wässrige Glyoxal-Lösung (40 Gew.-%), die mit einer kleinen Menge einer Lösung von p-Toluolsulfonsäure in Methanol versetzt wurde, über eine Pumpe eindosiert. Gleichzeitig wurde am untersten Boden der Kolonne gasförmiges Methanol eingespeist. Am Kopf der Kolonne wurde ein Destillat-Gemisch aus Wasser und Methanol aufgefangen, am Sumpfablauf wurde ein Gemisch aus 1,1,2,2-Tetramethoxyethan, 2,2-Dimethoxyacetaldehyd und Methanol aufgefangen. Das Kopfdestillat, bestehend aus Wasser und Methanol, wurde an einer weiteren Kolonne aufdestilliert, wobei das Methanol zurückgeführt, wieder verdampft und in den untersten Eintritt der Kolonne eingespeist wurde. Die Rückgewinnung des Methanols kann ebenfalls an der Reaktionskolonne geschehen, wenn oberhalb der Dosierstelle der Glyoxal-Lösung noch eine weiter Kolonne, die über einen Seitenabzug verfügt, eingesetzt wird. Das ablaufende Gemisch bestehend aus 1,1,2,2-Tetramethoxyethan (TME), 2,2-Dimethoxyacetaldehyd und Methanol wurde ebenfalls an einer weiteren Kolonne destillativ aufgearbeitet, wobei die leichtersiedenden Komponenten Methanol und 2,2-Dimethoxyacetaldehyd in den Prozess zurückgeführt wurden. Methanol wurde wiederum verdampft und am Boden der Kolonne eingespeist, 2,2-Dimethoxyacetaldehyd konnte der wässrigen Glyoxal-Lösung zugegeben und mit dieser am Kopf der Kolonne eingespeist werden. Die Glyoxal- und Methanol-Dosierung wurde so gewählt, dass Verhältnisse von Glyoxal zu Methanol von 1 mol : 4 mol bis zu 1 mol : 18 mol vorlagen. Die Kolonne wurde entweder unbeheizt gefahren oder bis zu einer Temperatur von 160 °C beheizt.

Die folgende Tabelle gibt die Ergebnisse unterschiedlicher Fahrweisen wieder:

| Nr | Verhältnis Glyoxal : MeOH [mol : mol] | Temp. [°C] | Dosierung Glyoxal [mol/h] | Reaktionszeit [h] | Ausbeute TME [g (%)] |
|---|---|---|---|---|---|
| 1 | 1 : 4 | 90 | 0,17 | 8 | 112(55%) |
| 2 | 1 : 8 | 90 | 0,17 | 8 | 138(68%) |
| 3 | 1 : 12 | 100 | 0,46 | 4 | 226 (82 %) |
| 4 | 1 : 12 | 90 | 0,25 | 8 | 276 (92 %) |
| 5 | 1 : 18 | 100 | 0,17 | 8 | 161 (79%) |
| 6 | 1 : 18 | 90 | 0,17 | 8 | 169(83%) |

Beispiel 2 (erfindungsgemäß):

Darstellung von 1,1,2,2-Tetramethoxyethan

Die unter Beispiel 1 beschriebene Apparatur wurde so betrieben, dass am Kopf der Kolonne 174 g eines Gemisches eindosiert wurden, welches aus 1,16 mol Glyoxal, 0,04 mol 2,2-Dimethoxyacetaldehyd, einer kleinen Menge p-Toluolsulfonsäure sowie aus Methanol und Wasser bestand. Gleichzeitig wurde am Boden der Kolonne gasförmiges Methanol eingeleitet.

Die Kolonne wurde beheizt. Nach der kompletten Dosierung wurde der Sumpfablauf neutralisiert und mittels Vakuumdestillation aufgearbeitet. Es wurden 162 g (1,08mol, 90 % bez. auf Glyoxal und 2,2-Dimethoxyacetaldehyd) 1,1,2,2-Tetramethoxyethan isoliert.

Beispiel 3 (erfindungsgemäß):

Darstellung von 1,1,2,2-Tetraethoxyethan

Die unter Beispiel 1 beschriebene Apparatur wurde so betrieben, dass am Kopf der Kolonne eine 40 %ige wässrige Glyoxal-Lösung, die mit einer kleinen Menge einer Lösung von p-Toluolsulfonsäure in Ethanol versetzt wurde, so eingespeist wurde, dass die Belastung 0,17 mol Glyoxal pro Stunde betrug. Am Boden der Kolonne wurde gasförmiges Ethanol mit einer Dosierung von 2 mol/h eingespeist. Die Kolonne wurde beheizt. Nach einer Reaktionszeit von 3 Stunden wurde der Sumpfablauf der Kolonne, der aus 1,1,2,2-Tetraethoxyethan, Ethanol und Spuren von 2,2-Diethoxyacetaldehyd bestand, destillativ aufgearbeitet, wobei sich 65 g (0,32 mol; 62 %) 1,1,2,2-Tetraethoxyethan isolieren ließen.

Beispiel 4 (erfindungsgemäß):

Darstellung von 1,1,2,2-Tetrapropoxyethan

Die unter Beispiel 1 beschriebene Apparatur wurde so betrieben, dass am Kopf der Kolonne eine 40 %ige wässrige Glyoxal-Lösung, die mit einer kleinen Menge einer Lösung von p-Toluolsulfonsäure in n-Propanol versetzt wurde, so eingespeist wurde, dass die Belastung 0,17 mol Glyoxal pro Stunde betrug. Am Boden der Kolonne wurde gasförmiges n-Propanol mit einer Dosierung von 2 mol/h eingespeist. Die Kolonne wurde beheizt. Nach einer Reaktionszeit von 2 Stunden wurde der Sumpfablauf der Kolonne, der aus 1,1,2,2-Tetrapropoxyethan, n-Propanol und ca. 1 % 2,2-Dipropoxyacetaldehyd bestand, mittels Vakuumdestillation aufgearbeitet, wobei sich 48 g (0,19 mol; 54 %) 1,1,2,2-Tetrapropoxyethan isolieren ließen.

Beispiel 5 (erfindungsgemäß):

Darstellung von 1,1,2,2-Tetramethoxyethan

In eine beheizbare 1m-Kolonne (Durchmesser 29 mm), gefüllt mit Kugeln mit einem Durchmesser von 5 mm des Katalysators KA-3 (Süd-Chemie) wurde am Kopf eine 40 %ige wässrige Glyoxal-Lösung mit einer Dosierrate von 0,17 mol/h eindosiert. Am Boden der Kolonne wurde gleichzeitig gasförmiges Methanol eingespeist. Während der gesamten Reaktion wurde die Apparatur unter Stickstoff als Schutzgas betrieben. Die Kolonne wurde beheizt. Am

Kopf der Kolonne wurde ein Gemisch aus Wasser und Methanol aufgefangen. Der Sumpfablauf der Kolonne bestand aus einem Gemisch aus 1,1,2,2-Tetramethoxyethan, 2,2-Dimethoxyacetaldehyd, 2-Hydroxyessigsäuremethyl-ester, 2-Hydroxyessigsäure und Methanol. Die Apparatur wurde 4 Stunden betrieben, der Sumpfablauf wurde gaschromatographisch untersucht. Gemäß Analyse lagen 11 g (0,07mol, 11 %) 1,1,2,2-Tetramethoxyethan vor.

Beispiel 6 (Vergleichsbeispiel):

Darstellung von 1,1,2,2-Tetramethoxyethan

In einem 4-1-Vierhalskolben mit Tropftrichter, Rührer und einer Kolonne, bei der Destillat über Kopf und über einen Seitenabzug entnommen werden konnte, wurden 100 g Methanol, 1 269 g (8,74 mol) einer 40 %igen wässrigen Glyoxal-Lösung und 70 g p-Toluolsulfonsäure vorgelegt. Aus diesem Ansatz wurde derart ein Gemisch aus Wasser und Methanol abdestilliert, dass über den Seitenabzug der Kolonne Wasser und Methanol und über Kopf reines Methanol entnommen wurde, welches dem Sumpf wieder zugeführt wurde. Die über den Seitenabzug entfernte Methanolmenge wurde per Gaschromatographie bestimmt und im Sumpf durch frisches Methanol ersetzt. Nach einer Reaktionszeit von 11 Stunden ließ sich im Sumpf die Bildung eines Gemisches bestehend aus 12,1 % 1,1,2,2-Tetramethoxyethan, 2,2-Dimethoxyacetaldehyd, Wasser, Methanol und einiger hochsiedender Komponenten nachweisen. Aus diesem Gemisch ließen sich destillativ 126 g 1,1,2,2-Tetramethoxyethan isolieren (0,84mol, 10 %).

## Patentansprüche

1. Verfahren zur Herstellung von Acetalen von α,β-Dicarbonylverbindungen der allgemeinen Formel $(R''O)_2CRCR'(OR'')_2$ (I), worin
R und R' unabhängig voneinander für H, (C1-C8)-Alkyl, (C3-C8)-cyclo-Alkyl, (C2-C8)-Alkenyl, (C2-C8)-Alkinyl oder (C6-C18)-Aryl und
R" unabhängig voneinander für (C1-C8-Alkyl), (C3-C8)-cyclo-Alkyl, (C2-C8)-Alkenyl oder (C2-C8)-Alkinyl oder für eine Kette X, wobei X die Bedeutung einer (C2-C12)-Alkylenkette oder (C2-C12)-Alkenylenkette hat, die die beiden Sauerstoffatome des α-Kohlenstoffatoms und/oder die beiden Sauerstoffatome des β-Kohlenstoffatoms miteinander verknüpft (Acetale (Ia)), steht,

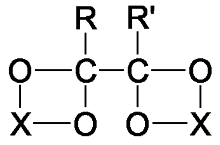

(Ia)

**dadurch gekennzeichnet,**
**dass** man Verbindungen des Typs R-CO-CO-R' mit Alkoholen des Typs R"OH oder HO-X-OH, wobei R, R', R" und X die zuvor genannte Bedeutung haben, kontinuierlich in einer Gegenstromapparatur zur Reaktion bringt.

2. Verfahren nach Anspruch 1,
**dadurch gekennzeichnet,**
**dass** die Verbindung des Typs R-CO-CO-R' in flüssiger Form oder in Form einer Lösung in die Gegenstromapparatur eingespeist wird und die Verbindung R"OH bzw. HO-X-OH in die Gegenstromapparatur eingespeist wird, derart, dass der Dampf des Alkohols R"OH beziehungsweise HO-X-OH der Verbindung des Typs R-CO-CO-R' in flüssiger Form oder in Form einer Lösung entgegen strömt.

3. Verfahren nach Anspruch 1,
**dadurch gekennzeichnet,**
**dass** die Verbindung R"OH beziehungsweise HO-X-OH in gasförmiger Form in die Gegenstromapparatur eingespeist wird, so dass die Verbindung R"OH beziehungsweise HO-X-OH gasförmig der Verbindung des Typs R-CO-CO-R' in flüssiger Form oder in Form einer Lösung entgegenströmt.

4. Verfahren nach Anspruch 1,
**dadurch gekennzeichnet,**

**dass** die Verbindung R-CO-CO-R' flüssig oder als Lösung und der Alkohol R"OH beziehungsweise HO-X-OH flüssig in die Gegenstromapparatur eingespeist werden und in der Apparatur eine Verdampfung des Alkohols erfolgt, derart, dass der so erzeugte Dampf des Alkohols R"OH beziehungsweise HO-X-OH der flüssigen α,β-Dicarbonylverbindungen R-CO-CO-R' oder deren Lösung entgegen strömt.

**5.** Verfahren nach Anspruch 1,
**dadurch gekennzeichnet,**
**dass** als Gegenstromapparatur ein Dünnschichtverdampfer, ein Fallfilmverdampfer, eine Reaktionskolonne oder eine Rührkesselkaskade eingesetzt wird.

**6.** Verfahren nach Anspruch 1,
**dadurch gekennzeichnet,**
**dass** die Reaktion in Abwesenheit oder in Gegenwart eines sauren oder eines basischen Katalysators durchgeführt wird.

**7.** Verfahren nach Anspruch 1,
**dadurch gekennzeichnet,**
**dass** die Reaktion in Anwesenheit eines homogenen oder heterogenen Katalysators durchgeführt wird.

**8.** Verfahren nach Anspruch 1,
**dadurch gekennzeichnet,**
**dass** die Reaktion bei einer Temperatur von 20 bis 250 °C durchgeführt wird.

**9.** Verfahren nach Anspruch 1,
**dadurch gekennzeichnet,**
**dass** die Reaktion bei einem Druck von 20 mbar bis 20 bar durchgeführt wird.

**10.** Verfahren nach Anspruch 1,
**dadurch gekennzeichnet,**
**dass** das molare Verhältnis von eingespeister Menge α,β-Dicarbonylverbindung R-CO-CO-R' zur Menge des eingespeisten Alkohols R"OH 1 : 2 bis 1 : 2000 beträgt.

**11.** Verfahren nach Anspruch 1,
**dadurch gekennzeichnet,**
**dass** das molare Verhältnis von eingespeister Menge α,β-Dicarbonylverbindung R-CO-CO-R' zur Menge des eingespeisten Alkohols R"OH 1 : 8 bis 1 : 20 beträgt.

**12.** Verfahren nach Anspruch 1,
**dadurch gekennzeichnet,**
**dass** das molare Verhältnis von eingespeister Menge α,β-Dicarbonylverbindung R-CO-CO-R' zur Menge des eingespeisten Alkohols HO-X-OH 1 : bis 1 : 1000 beträgt.

**13.** Verfahren nach Anspruch 1,
**dadurch gekennzeichnet,**
**dass** das molare Verhältnis von eingespeister Menge α,β-Dicarbonylverbindung R-CO-CO-R' zur Menge des eingespeisten Alkohols HO-X-OH 1 : 4 bis 1 : 10 beträgt.

**14.** Verfahren nach Anspruch 1,
**dadurch gekennzeichnet,**
**dass** die bei der Reaktion auftretenden Nebenströme mittels Destillation isoliert und in den Prozess zurückgeführt werden.

Europäisches Patentamt

# EUROPÄISCHER RECHERCHENBERICHT

Nummer der Anmeldung
EP 04 10 0262

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | US 2 360 959 A (LOUIS G MACDOWELL ET AL) 24. Oktober 1944 (1944-10-24) * Anspruch 1 * ----- | 1 | C07C41/56 C07C41/58 |
| D,A | JONATHAN M KLIEGMAN ET AL: "Glyoxal Derivatives. V. Reaction of Alcohols with Glyoxal" JOURNAL OF ORGANIC CHEMISTRY., Bd. 38, Nr. 3, 1973, Seiten 556-560, XP002279286 USAMERICAN CHEMICAL SOCIETY. EASTON. * Seite 557, Spalte 1; Tabelle 1 * ----- | 1 | |

RECHERCHIERTE SACHGEBIETE (Int.Cl.7)
C07C

Der vorliegende Recherchenbericht wurde für alle Patentansprüche erstellt

| Recherchenort | Abschlußdatum der Recherche | Prüfer |
|---|---|---|
| Den Haag | 7. Mai 2004 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 04 10 0262

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-05-2004

| Im Recherchenbericht angeführtes Patentdokument | Datum der Veröffentlichung | Mitglied(er) der Patentfamilie | Datum der Veröffentlichung |
|---|---|---|---|
| US 2360959 A | | KEINE | |

EPO FORM P0461

Für nähere Einzelheiten zu diesem Anhang : siehe Amtsblatt des Europäischen Patentamts, Nr.12/82